# NEW EUROPEAN PATENT SPECIFICATION

(11) **EP 0 369 803 B2**
(45) Date of publication and mention of the opposition decision: **14.08.1996**
(45) Mention of the grant of the patent: 31.03.1993
(21) Application number: 89311906.5
(22) Date of filing: 16.11.1989
(51) Int. Cl.: C07C 317/46, C07C 317/24, C07C 323/22, C07C 323/32, C07C 69/76, C07C 63/04

(54) **Trisubstituted benzoic acid intermediates**
Trisubstituierte Benzoesäuren als Zwischenverbindungen
Produits intermédiaires du type acide benzoique trisubstitué

(30) Priority: 18.11.1988 US 273391
(43) Date of publication of application: 23.05.1990
(73) Proprietor: ZENECA INC., Wilmington, Delaware 19897 (US)
(72) Inventor: Michaely, William J., El Cerrito California 94530 (US); Curtis, Jeff K., Berkeley California 94708 (US)
(74) Representative: Waterman, John Richard

(56) References cited:
- EP-A- 352 543
- EP-A- 0 195 247
- US-A- 4 780 127
- US-A- 4 816 066

## Description

Certain 2-(2'3'4'tri-substituted benzoyl)-1,3-cyclohexanedione herbicides are described in U.S. Patent 4,780,127, issued October 25, 1988 and U.S. Patent No. 4,816,066 filed December 4, 1987 incorporated herein by reference. In addition compounds are described in U S application entitled 2-(2',3',4'-trisubstituted benzoyl)-1,3-cyclohexanediones, with William J Michaely, inventor, filed herwith, and summarised hereinafter. The above-described herbicidal compounds can have the following structural formula 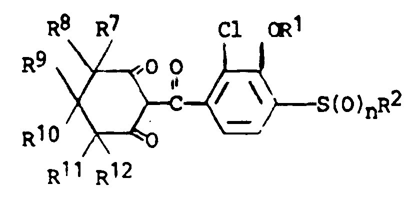
wherein R⁷ through R¹² are hydrogen or C₁-C₄ alkyl; R' is C₁-C₄ alkyl, C₁-C₄ haloalkyl, CH₂CH₂-OCH₃, CH₂CH₂OC₂H₅, CH₂CH₂SCH₃, or CH₂CH₂SC₂H₅; R² is C₁-C₄ alkyl; and n is the integer 0 or 2.

These herbicides can be prepared by reacting a dione of the structural formula 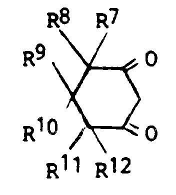
wherein R⁷ through R¹² are as defined with a mole of trisubstituted benzoyl chloride of the structural formula 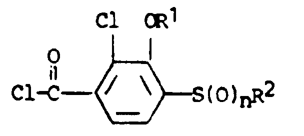
wherein n, R¹ and R² are as defined above.

Certain alkylsulphonyl benzoic acids useful as intermediates in the synthesis of pyrazole herbicides are disclosed in EP-A-352543.

### Description of the Invention

This invention has several embodiments which are as follows:
Embodiment A relates to novel intermediate compounds having the structural formula 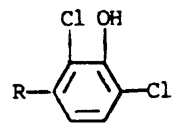
wherein R is cyano; carboxy; or -CO₂Rₐ where Rₐ is C₁-C₄ alkyl, preferably ethyl; most preferably R is - CO₂ C₂H₅.

Embodiment B relates to novel intermediate compounds having the structural formula 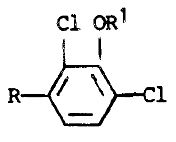
wherein R is cyano; carboxy or -CO₂Rₐ wherein Rₐ is C₁-C₄ alkyl, preferably ethyl, most preferably R is - CO₂ C₂H₅ and R¹ is C₁-C₄ alkyl; preferably C₁-C₂ alkyl; C₁-C₄ haloalkyl; -CH₂CH₂OCH₃; -CH₂CH₂OC₂H₅; - CH₂ CH₂SCH₃ or -CH₂CH₂SC₂H₅, with the proviso that when R is carboxy, then R¹ is -CH₂CH₂OCH₃; -CH₂ CH₂OC₂H₅; -CH₂CH₂SCH₃ or -CH₂CH₂SC₂H₅.

Embodiment C relates to novel intermediate compounds having the structural formula 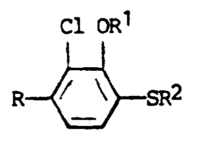
wherein R is cyano; carboxy or -CO₂Rₐ wherein Rₐ is C₁-C₄ alkyl, preferably ethyl, most preferably R is CO₂C₂H₅; R¹ is C₁-C₄ alkyl; C₁-C₄ haloalkyl; -CH₂CH₂OCH₃, -CH₂CH₂OC₂H₅, -CH₂CH₂SCH₃ or -CH₂CH₂SC₂H₅ and R² is C₁-C₄ alkyl, preferably methyl, ethyl or n-propyl with the proviso that when R is carboxy, then R¹ is-CH₂CH₂OCH₃, -CH₂CH₂OC₂H₅, -CH₂CH₂SCH₃ or -CH₂CH₂SC₂H₅.

Embodiment D relates to novel intermediate compounds having the structural formula 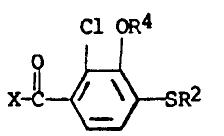
wherein X is chlorine or hydroxy; R⁴ is -CH₂CH₂OCH₃, -CH₂CH₂OC₂H₅, -CH₂CH₂SCH₃ or -CH₂CH₂SC₂H₅, preferably -CH₂CH₂OCH₃ or -CH₂CH₂OC₂H₅; and R² is C₁-C₄ alkyl, preferably methyl, ethyl or n-propyl.

Embodiment E relates to novel intermediate compounds having the structural formula 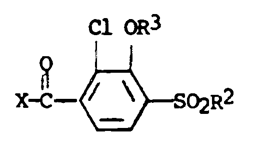
wherein X is hydroxy or chlorine; R³ is -CH₂CH₂OCH₃ or -CH₂CH₂OC₂H₅ and R² is C₁-C₄ alkyl, preferably methyl, ethyl or n-propyl, provided that when X is hydroxy, R² is not methyl.

Embodiment F relates to novel intermediate compounds having the structural formula 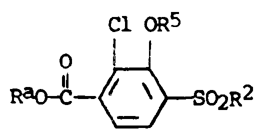
wherein R^{a} is C₁-C₄ alkyl, preferably ethyl, R⁵ is C₁-C₄ alkyl; C₁-C₄ haloalkyl; -CH₂CH₂OCH₃ or - CH₂CH₂OC₂H₅; preferably -CH₂CH₂OCH₃ or -CH₂CH₂OC₂H₅ and R² is C₁-C₄ alkyl, preferably methyl, ethyl or n-propyl.

In embodiments A-C, the group R can also be trifluoromethyl.

The several intermediate compounds of this invention can be prepared by the general method shown in the Figure of the next page with R, R¹, R² and R⁵ being as defined. The group R^{z} is C₁-C₄ alkyl.

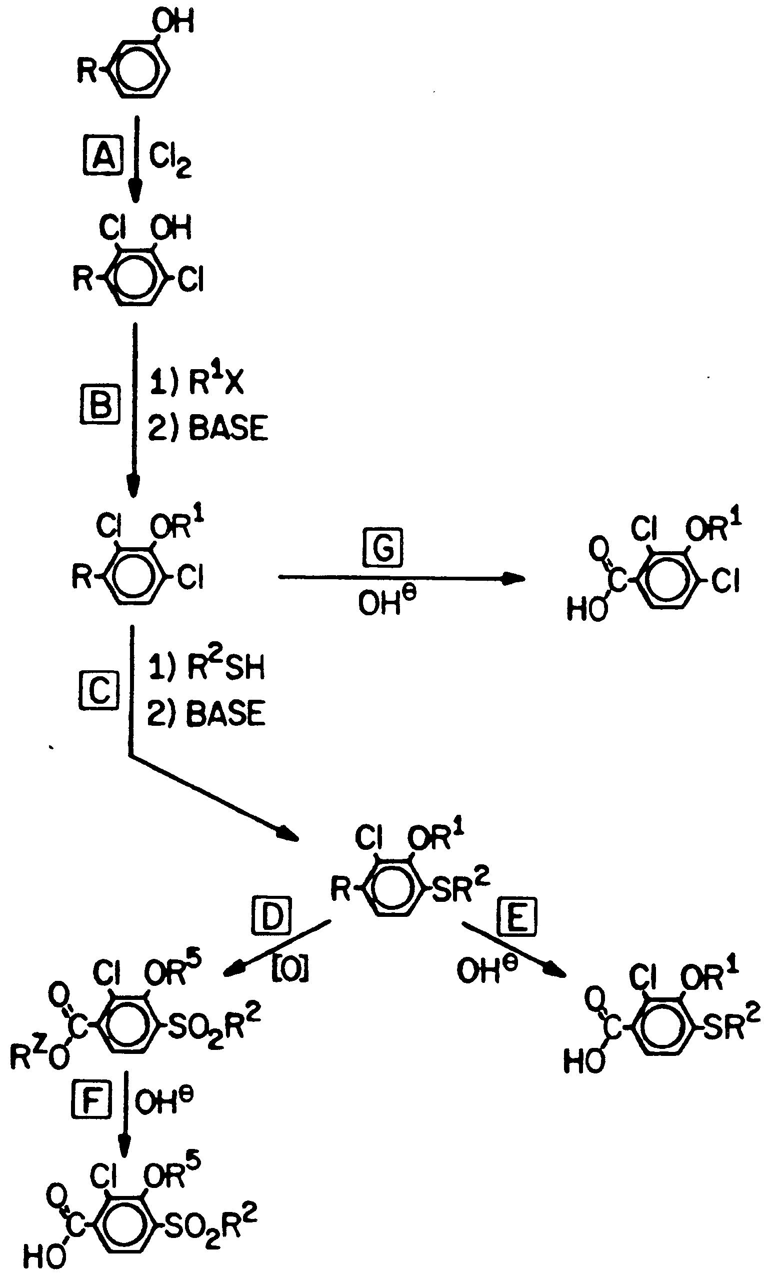

Referring to the Figure, and particularly to Reaction Steps (A) through (G), consider the following:

Generally in Reaction Step (A), a mole amount of the 3-substituted phenol is reacted with 2 moles of chlorine and a catalytic amount of a C₁-C₁₀ alkylamine, preferably tert-butylamine or diisopropylamine in a solvent such as methylene chloride, at a temperature between -70° to 70°C. After this reaction, the free chlorinated phenol is isolated by normal procedures.

For Reaction Step (B), one mole of the dichloro-substituted phenol reaction product of Step (A) is reacted with an appropriate alkylating agent such as a 2-chloroethyl ethyl ether, 2-chloroethyl methyl ether, 2-chloroethyl methyl sulfide, 2-chloroethyl ethyl sulfide or C₁-C₄ alkylchloride along with a catalytic amount of potassium iodide and a mole excess of a base such as potassium carbonate. Alkyl iodides such as methyl iodide or ethyl iodide may also be used. In these cases the catalytic potassium iodide is not needed and little or no heat is required. The reaction is run at 25°C to 80°C for 4 hours with agitation. The reaction product is recovered by conventional techniques.

For Reaction Step (C), the dichloro compound from Reaction Step (B) is reacted with an equal mole amount of a C₁-C₄ alkyl mercaptan along with a mole excess of a base such as potassium carbonate in a solvent such as dimethylformamide. The reaction is run for several hours at a temperature between 50°C to 100°C with stirring underan inert atmosphere such as nitrogen. The desired reaction product is recovered by conventional techniques.

For Reaction Step (D) a mole amount of the alkyl ester of 2-chloro-4-alkylthio benzoic compound is oxidized with at least 3 moles of an oxidizing agent such as m-chloroperbenzoic acid in a suitable solvent such as methylene chloride by stirring a solution of the reactants at 20° to 100°C. The desired intermediate is recovered by conventional techniques. During this reaction step the 4-alkylthio substituent is oxidized to the corresponding alkylsulfone.

For Reaction Step (E) a mole amount of the 2-chloro-3-substituted-4-alkylthio ester or cyano compound is hydrolyzed with a base such as sodium hydroxide to the corresponding 2-chloro-3-substituted-4-alkylthio benzoic acid. The hydrolysis is run in a solvent such as an 80 percent methanol-water mixture. The reaction can be run at 25°-100°C with stirring. The desired product is recovered by conventional techniques.

For Reaction Step (F) the alkyl ester of the trisubstituted benzoic acid is converted to the trisubstituted benzoic acid by the hydrolysis step taught in Reaction Step (E)

In the alternative, the tri-substituted benzoic acid reaction product of Reaction Step (F) can be directly prepared from the reaction product of Reaction Step (C) by a combination hydrolysis of the 2-chloro-3-substituted-4-alkylthio ester or cyano compound to the corresponding benzoic acid and an oxidation of the 4-alkylthio substituent to the corresponding 4-alkylsulfone. The hydrolysis and oxidation steps can be jointly carried out by reacting a mole of the ester or cyano compound with at least 5 moles of sodium or calcium hypochlorite in a suitable solvent such as dioxane-water, by heating a solution of the reactants from about 25°C to about 100°C, followed by the acidification with concentrated hydrochloric acid. Filtration of the resulting precipitate yields the desired product.

For Reaction Step (G) the dichloro compound from Reaction Step (B) is converted to the benzoic acid by the hydrolysis step taught in Reaction Step (E).

The intermediate benzoic acids described herein can easily be converted to their respective acid chlorides and then to their acid cyanides, if desired, by the following two reactions. First, a mole of oxalyl chloride and a catalytic amount of dimethylformamide in a suitable solvent such as methylene chloride at a temperature of 20 to 40°C for 1 to 4 hours is heated with a mole of the intermediate benzoic acid. The corresponding benzoic acid cyanide can easily be prepared from the benzoic acid chloride by reaction with cuprous cyanide at a temperature of 50° to 220°C for 1 to 2 hours.

The trisubstituted benzoic acid chloride intermediates are useful in the preparation of the previously described herbicidal 2-(2',3',4'-trisubstituted benzoyl)-1,3-cyclohexanediones.

The following series of examples teach the synthesis of representative intermediate compounds of this invention. The structures of all compounds of the examples and tables were verified by nuclear magnetic resonance (NMR), infrared spectroscopy (IR) and mass spectroscopy (MS).

### EXAMPLE 1

### Ethyl 2,4-chloro-3-hydroxybenzoate

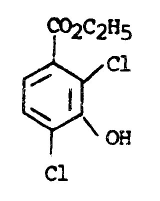

In a 3-neck, 1-liter flask equipped with a mechanical stirrer, condenser, thermometer and a diffusion tube was added a solution of 106 grams (0.64 mole) ethyl 3-hydroxybenzoate and 0.5 grams diisopropylamine in 600 milliliters (ml) dichloroethane at reflux. Chlorine (112 grams, 1.6 mole) was added through the diffusion tube over a period of 6 hours then let cool to room temperature. After cooling, the solution was washed with 200 ml 5% sodium bisulfite solution, then with 200 ml water, dried (MgSO₄) and reduced under vacuum. Yield was 151 g of an oil. This mixture of chlorinated compounds (66% above product) can be recrystallized in ether/pentane by cooling to -20°C to give pure ethyl 2,4-dichloro-3-hydroxybenzoate. The structure of this compound and all examples were verified by nuclear magnetic resonance (NMR), infrared spectroscopy (IR) and mass spectroscopy (MS).

Additional compounds were prepared by the same procedure as described in Example 1 and are listed in Table 1.

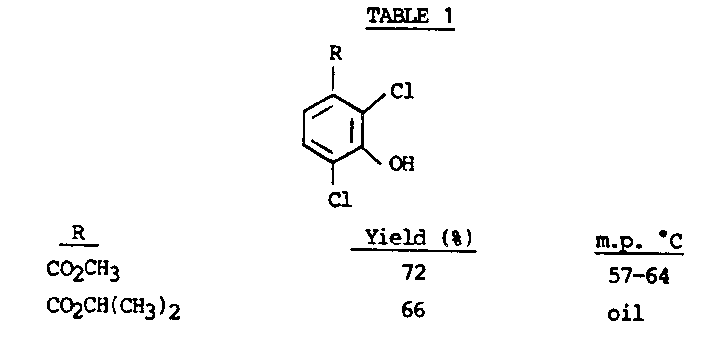

### EXAMPLE 2

### Ethyl 2,4-chloro-3-(2-methoxyethoxy) benzoate

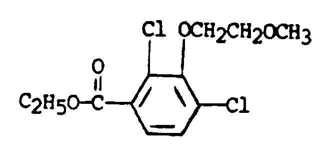

A solution of 18 g (77 millimoles, mmol) ethyl 2,4-dichloro-3-hydroxybenzoate, 22 g (3 equivalents) (eq) 2-chloroethyl methyl ether, 22 g (2eq) potassium carbonate and ca. 0.5g sodium iodide in 100 ml DMF was heated at 80°C for 1.5 hours. To the cooled solution was added 400 ml ether. The organic phase was washed with 100 ml water (2 times), 100 ml 100% NaOH and 100 ml 10% HCl. Dried (MgSO₄) and reduced under vacuum. The yield was 20 g (68 mmole).

Additional compounds were prepared by the same procedure as described in Example 2 (except in those cases using alkyl iodide then the potassium iodide catalyst was omitted and little or no heat is required) and are listed in Table 2.

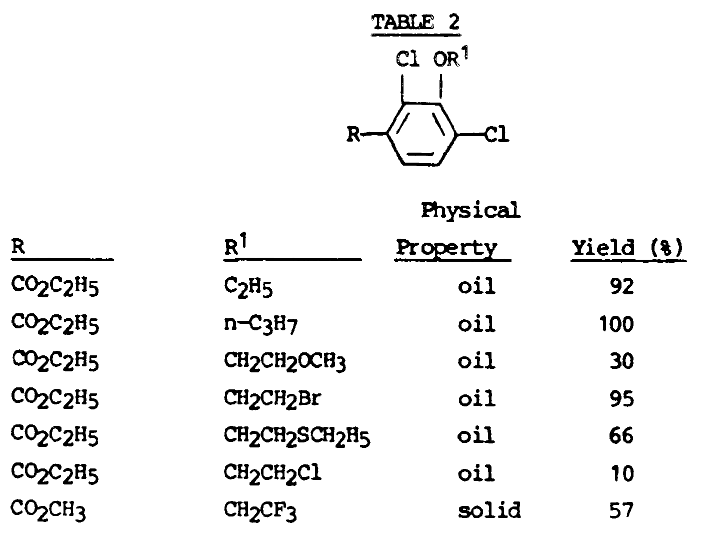

### EXAMPLE 3

### Ethyl 2-chloro-3-(2-methoxyethoxy)-4-ethylthiobenzoate

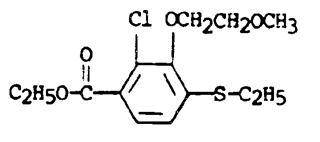

A solution of 10 g (34 mmole) ethyl 2,4-dichloro-3-(2-methoxyethoxy)benzoate, 10 g (4 eq) ethanethiol and 10 g (2 eq) potassium carbonate in 100 ml DMF was heated (approximately 100°C) for 2 hours, then let cool overnight. Added 400 ml diethyl ether and washed with 100 ml water (two times), 100 ml 10% HCl and 100 ml 10% NaOH. Dried (MgSO₄) and reduced under vacuum. Yield 10 g (31 mmole) of an oil.

Additional compounds were prepared by the same procedure as described in Example 3 and are listed in Table 3.

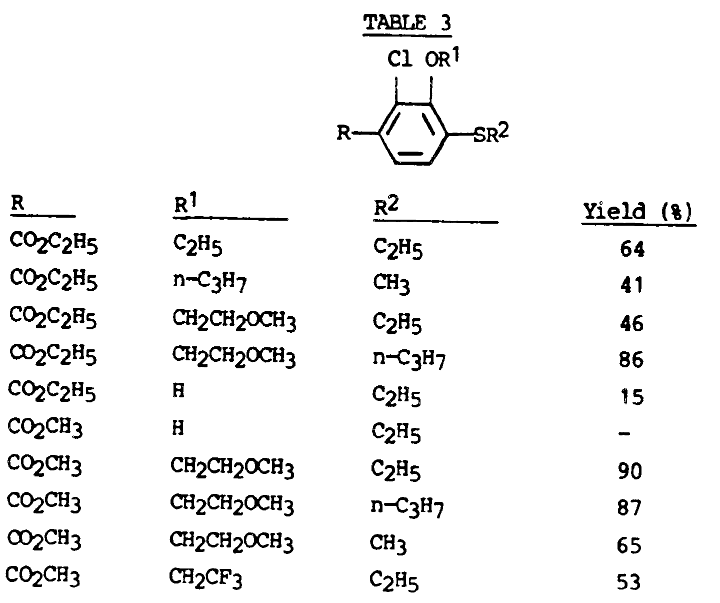

### EXAMPLE 4

### Ethyl 2-chloro-3-(2-methoxyethoxy)-4-ethylsulfonyl benzoate

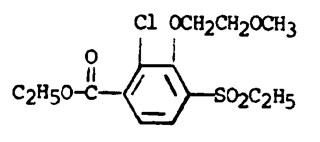

The ester, ethyl 2-chloro-3-(2-methoxyethoxy)-4-ethylthiobenzoate from Example 3 (10 g) was dissolved in 100 ml of methylene chloride and cooled with an ice bath. Next 18 g solid m-chloroperoxybenzoic acid (85% pure, 2.2 equivalents) was added in portions over a period of 2 hours. The crude reaction mixture was allowed to warm to room temperature. After 1 hour at room temperature the excess peracid was destroyed with sodium bisulfite (100 ml 5% solution). The organic layer was washed two times with 5% sodium hydroxide (100%) and stripped under vacuum to give 11.3 grams of pure ethyl 2-chloro-3-(2-methoxyethoxy)-4-ethylsulfonylbenzoate as a viscous oil.

Additional compounds were prepared by the same procedure as described in Example 4 and are listed in Table 4.

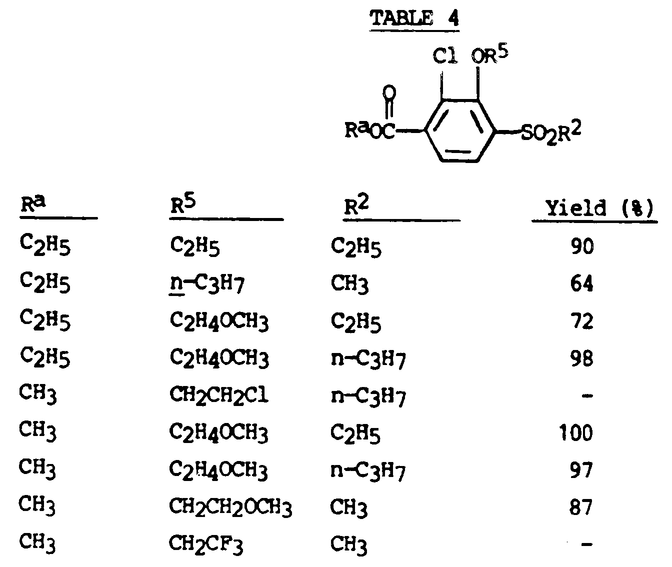

### EXAMPLE 5

### 2-chloro-3-(2-methoxyethoxy)-4-ethylsulfonylbenzoic acid

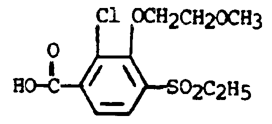

To 11.3 g (0.03 mole) of the ethyl 2-chloro-3-(2-methoxyethoxy)-4-ethylsulfonylbenzoate in 100 ml of 96% ethanol was added dropwise 16 ml (1.2 eq) of 10% sodium hydroxide. After stirring at room temperature for 4 hours, 100 ml of diethyl ether was added and the organic phase was extracted with 50 ml of 5% NaOH. The aqueous phase was acidified with 10% HCl and extracted two times with 50 ml chloroform. The organic phase was dried with MgSO₄ and concentrated under vacuum to yield 8.8 grams of 2-chloro-3-(2-methoxyethoxy)4-ethylsulfonylbenzoic acid as viscous oil.

An additional compound was prepared by the same procedure as described in Example 5 and is listed in Table 5.

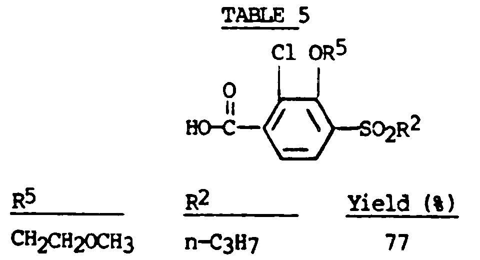

### EXAMPLE 6

### 2-Chloro-3-(2-methoxyethoxy)-4-ethylthio benzoic acid

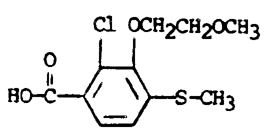

Three grams (8.2 mmol) ethyl 2-chloro-3-(2-methoxyethoxy)-4-propane-thiobenzoate was dissolved in 20 ml 96% ethyl alcohol. To this was added 3.9 ml 10% sodium hydroxide in water. After stirring 4 hours at room temperature, 100 ml of diethyl ether was added to the solution. The solution was extracted twice with 50 ml 5% sodium hydroxide solution. The combined caustic extracts were acidified with 10% hydrochloric acid and extracted twice with 50 ml portions of chloroform. The chloroform extracts were dried over magnesium sulfate and the chloroform removed in vacuo to afford the free acid (2.0 g, 72%) as a soft solid.

Additional compounds were prepared by the same procedure as described in Example 6 and are listed in Table 6.

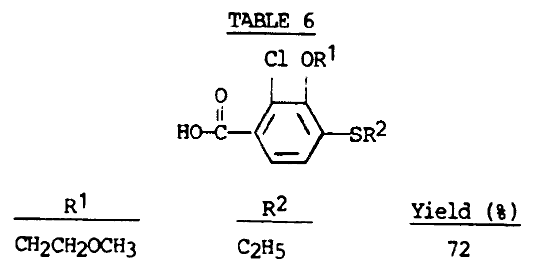

### EXAMPLE 7

### 2,4-Dichloro-3-(2-methoxyethoxy) benzoic acid

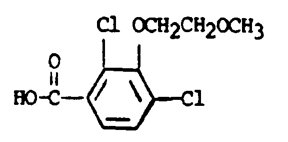

Sixteen grams (41 mmol) ethyl 2,4-dichloro-3-(2-methoxyethoxy)benzoate was dissolved in 100 ml of 96% ethanol. To this was added, in portions, 18 ml (ca. 1.1 equivalents) 10% sodium hydroxide. After stirring 4 hours at room temperature, 250 ml diethyl ether was added to the solution. The solution was extracted twice with 50 ml 5% sodium hydroxide. The combined caustic extracts were acidified with a 10% hydrochloric acid solution and extracted twice with 75 ml portions of chloroform. The chloroform extracts were dried (magnesium sulfate) and the chloroform removed in vacuo to afford the free acid (12.8 g, 79%) as a white solid.

Additional compounds were prepared by the same procedures as described in Example 7 and are listed in Table 7.

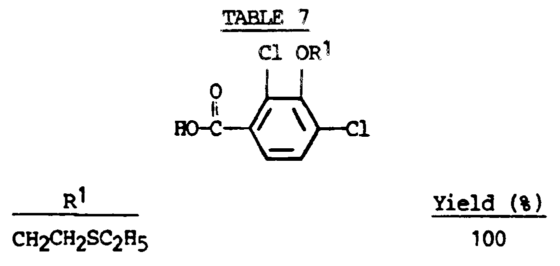

The above-described benzoic acids can be readily converted to their acid chlorides using oxalyl chloride and a catalytic amount of dimethylformamide. These acid chlorides can be reacted with the above-described, 1,3-cyclohexanedione to prepare the above-described herbicidal 2,3,4-trisubstituted benzoyl-1,3-cyclohexanediones according to the following two-step reaction.

The process proceeds via the production of an enol ester intermediate as shown in reaction (1). The final product is obtained by rearrangement of the enol ester as shown in reaction (2). The two reactions may be conducted as separate steps by isolation and recovery of the enol ester using conventional techniques prior to conducting step (2), or by addition of a cyanide source to the reaction medium after the formation of the enol ester, or in one step by inclusion of the cyanide source at the start of reaction (1). 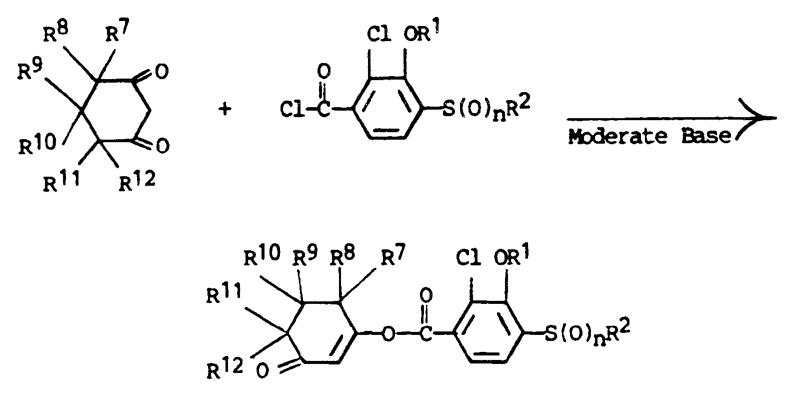
wherein n and R¹, R² and R⁷ through R¹² are as defined above and the moderate base is such as tri-C₁-C₆ alkylamine, pyridine, alkali metal carbonate or alkali metal phosphate.

Generally, in step (1) mole amounts of the dione and substituted benzoyl chloride are used, along with a slight mole excess of a moderate base. The two reactants are combined in an organic solvent such as acetonitrile, methylene chloride, toluene, ethyl acetate or dimethylformamide. The base and benzoyl reactant preferably are added to the reaction mature with cooling. The mixture is stirred at 0°C-50°C until the reaction is substantially complete.

The reaction is worked up by conventional techniques. 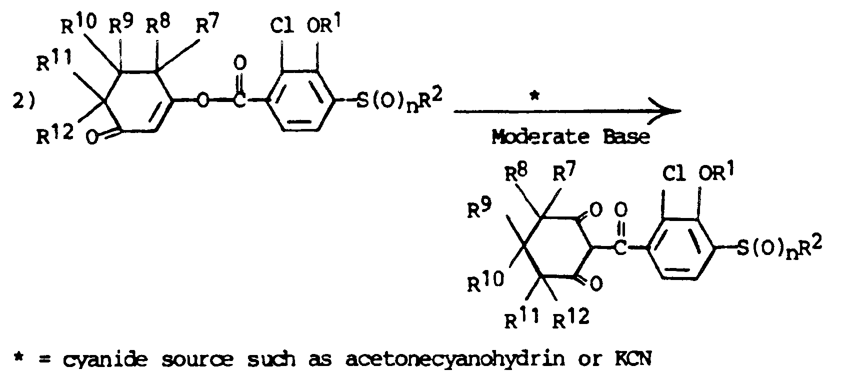
wherein R¹, R² and R⁷ through R¹² are as defined above.

Generally, in step (2) a mole of the enol ester intermediate is reacted with 1 to 4 moles of the base, preferably about 2 moles of moderate base and from 0.01 mole to about 0.5 mole or higher, preferably around 0.1 mole of the cyanide source (e.g., potassium cyanide or acetone cyanohydrin). The mixture is stirred in a reaction pot until the rearrangement is substantially complete at a temperature below 80°C, preferably about 20°C to about 40°C, and the desired product is recovered by conventional techniques.

The term "cyanide source" refers to a substance or substances which under the rearrangement conditions consists of or generates hydrogen cyanide and/or cyanide anion.

The process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium and potassium cyanide; cyanohydrins of methyl alkyl ketones having from 1-4 carbon atoms in the alkyl groups, such as acetone or methyl isobutyl ketone cyanohydrins; cyanohydrins of benzaldehyde or of C₂-C₅ aliphatic aldehydes such as acetaldehyde, propionaldehyde, etc., cyanohydrins; zinc cyanide; and hydrogen cyanide itself. Hydrogen cyanide is considered most advantageous as it produces relatively rapid reaction and is inexpensive. Among cyanohydrins the preferred cyanide source is acetone cyanohydrin.

The cyanide source is used in an amount up to about 50 mole % based on the enol ester. It may be used in as little as about 1 mole % to produce an acceptable rate of reaction at about 40°C on a small scale. Larger scale reactions give more reproducible results with slightly higher catalyst levels of about 2 mole %. Generally about 1-10 mole % of the cyanide source is preferred.

The process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalysts are the Crown ethers.

A number of different solvents may be usable in this process, depending on the nature of the acid chloride or the acylated product. A preferred solvent for this reaction is 1,2-dichloroethane. Other solvents which may be employed depending on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperatures up to about 50°C.

As mentioned previously a U S application of William J Michaely describes compounds which can be prepared from the intermediates of the present applications.

This application relates to 2-(2',3',4'-trisubstituted benzoyl)-1,3-cyclohexanediones, their use as nerbicides and herbicidal compositions containing the compounds.

Embodied within the scope of this application are novel compounds having the following structural formula 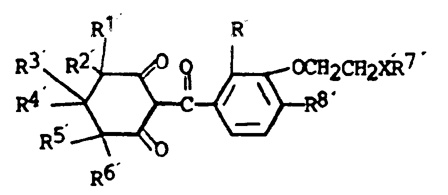
wherein
X' is oxygen or sulfur, preferably oxygen;
R' is chlorine or bromine;
R^{1'} is hydrogen or C₁-C₄ alkyl, preferably methyl;
R^{2'} is hydrogen or C₁-C₄ alkyl, preferably methyl;
R^{3'} is hydrogen or C₁-C₄ alkyl, preferably methyl;
R^{4'} is hydroxy, hydrogen or C₁-C₄ alkyl, preferably methyl; or
R^{3'} and R^{4'} together are carbonyl (=0) with the proviso that R¹,
R^{2'}, R^{5'} and R^{6'} and C₁-C₄ alkyl, preferably all methyl;
R^{5'} is hydrogen or C₁-C₄ alkyl, preferably methyl;
R^{6'} is hydrogen, C₁-C₄ alkyl, preferably methyl, C₁-C₄ alkylthio, preferably methylthio or C₁-C₄ alkylsulfonyl, preferably methylsulfonyl, with the proviso that when R⁶ is C₁-C₄ alkylthio or C₁-C₄ alkylsulfonyl, the R^{3'} and R^{4'} are not together carbonyl;
R^{7'} is methyl or ethyl; and
R^{8'} is (1) halogen, preferably chlorine or bromine; (2) nitro; or (3) R^{b}SOₙ- wherein n is the integer 0 or 2, preferably 2, and R^{b} is (a) C₁-C₃ alkyl, preferably methyl or ethyl.

The term "C₁-C₄ alkyl" includes methyl, ethyl, n-propyl, iso-propyl, n-butyl, sec-butyl, isobutyl and t-butyl. The term "halogen" includes chlorine, bromine, iodine and fluorine. The term "C₁-C₄ alkoxy" includes methoxy, ethoxy, n-propoxy, isopropoxy, n-butoxy, sec-butoxy, isobutoxy and t-butoxy. The term "haloalkyl" includes the eight alkyl groups with one or more hydrogens replaced by chloro, bromo, iodo or fluoro.

Salts of the above-described compounds (as defined hereinafter) are also the subject of the application.

The compounds of this application can have the following four structural formulae because of tautomerism: 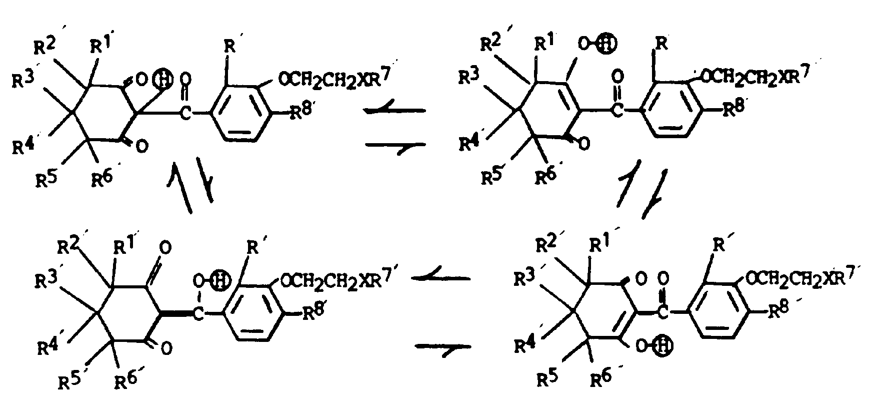
wherein X', R', R^{1'}, R^{2'}, R^{3'}, R^{4'}, R^{5'}, R^{6'}, R^{7'} and R^{8'} are as defined above.

The circled proton on each of the four tautomers is reasonably labile. These protons are acidic and can be removed by any base to give a salt having an anion of the following four resonance forms: 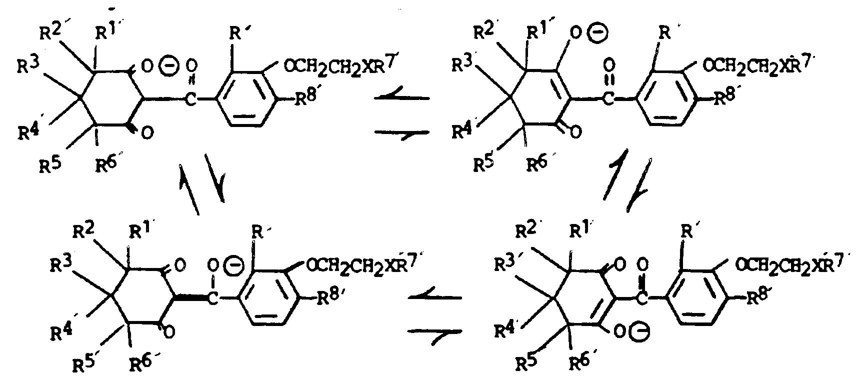
wherein X', R', R^{1'}, R^{2'}, R^{3'}, R^{4'}, R^{5'}, R^{6'}, R^{7'} and R^{8'} are as defined above.

Examples of cations of these bases are inorganic cations such as alkali metals e.g. lithium, sodium, and potassium or organic cations such as substituted ammonium, sulfonium or phosphonium wherein the substituent is an aliphatic or aromatic group.

The compounds of this invention and their salts are active herbicides of a general type. That is, they are herbicidally effective against a wide range of plant species. The method of controlling undesirable vegetation of the present invention comprises applying an herbicidally effective amount of the above-described compounds to the area where control is desired.

The compounds of the said application can be prepared by the following two-step general method.

The process proceeds via the production of an enol ester intermediate as shown in reaction (1). The final product is obtained by rearrangement of the enol ester as shown in reaction (2). The two reactions may be conducted as separate steps by isolation and recovery of the enol ester using conventional techniques prior to conducting step (2), or by addition of a cyanide source to the reaction medium after the formation of the enol ester, or in one step by inclusion of the cyanide source at the start of reaction (1). 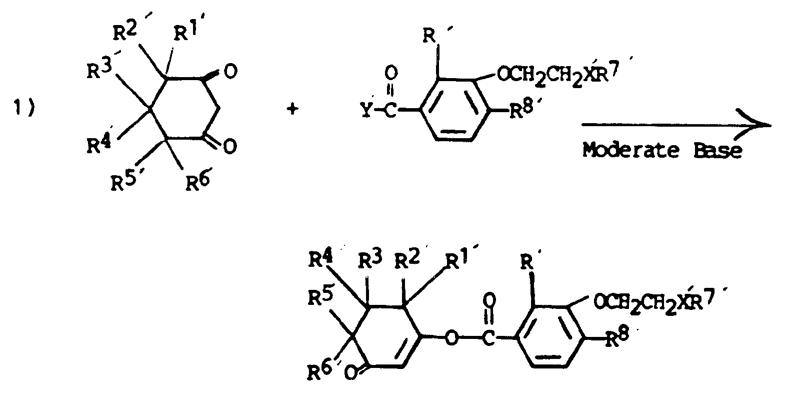
wherein X' and R' through R^{8'} are as defined and Y' is halogen, preferably chlorine, C₁-C₄ alkyl-C(O)-O-, C₁-C₄ alkoxy-C(O)-O- or 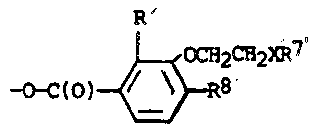
wherein X', R', R^{7'} and R^{8'} in this portion of the molecule are identical with those in the reactant shown above and the moderate base is as defined, preferably tri-C₁-C₆ alkylamine, pyridine, alkali metal carbonate or alkali metal phosphate.

Generally, in step (1) mole amounts of the dione and substituted benzoyl reactant are used, along with a mole amount or excess of the base. The two reactants are combined in an organic solvent such as methylene chloride, toluene, ethyl acetate or dimethylformamide. The base of benzoyl reactant preferably are added to the reaction mixture with cooling. The mixture is stirred at 0°C-50° until the reaction is substantially complete.

The reaction product is worked up by conventional techniques. 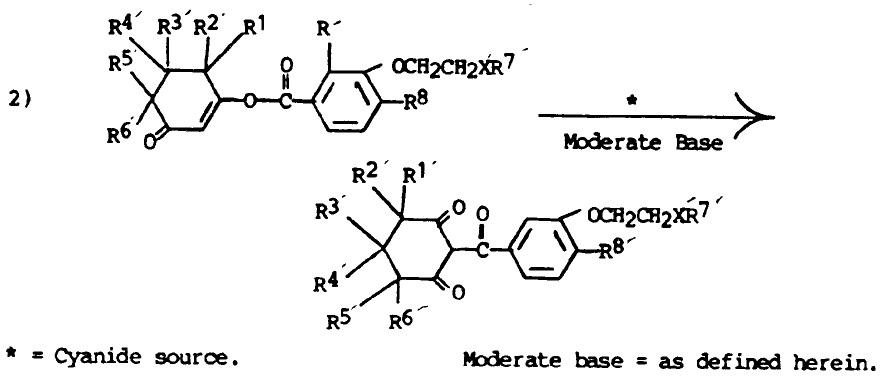
wherein X' and R' through R^{8'} are as defined.

Generally, in step (2) a mole of the enol ester intermediate is reacted with 1 to 4 moles of the base, preferably about 2 moles of moderate base and from 0.01 mole to about 0.5 mole or higher, preferably around 0.1 mole of the cyanide source (e.g., potassium cyanide or acetone cyanohydrin). The mixture is stirred in a reaction pot until the rearrangement is substantially complete at a temperature below 80°C, preferably about 20°C to about 40°C, and the desired product is recovered by conventional techniques.

The term "cyanide source" refers to a substance or substances which under the rearrangement conditions consists of or generates hydrogen cyanide and/or cyanide anion.

The process is conducted in the presence of a catalytic amount of a source of cyanide anion and/or hydrogen cyanide, together with a molar excess, with respect to the enol ester, of a moderate base.

Preferred cyanide sources are alkali metal cyanides such as sodium and potassium cyanide; cyanohydrins of methyl alkyl ketones having from 1-4 carbon atoms in the alkyl groups such as acetone or methyl isobutyl ketone cyanohydrins; cyanohydrins of benzaldehyde or of C₂-C₅ aliphatic aldehydes such as acetaldehyde, propionaldehyde, etc., cyanohydrins; zinc cyanide; tri(lower alkyl) silyl cyanides, notably trimethyl silyl cyanide; and hydrogen cyanide itself. Hydrogen cyanide is considered most advantageous as it produces relatively rapid reaction and is inexpensive. Among cyanohydrins the preferred cyanide source is acetone cyanohydrin.

The cyanide source is used in an amount up to about 50 mole percent based on the enol ester. It may be used in as little as about 1 mole percent to produce an acceptable rate of reaction at about 40°C on a small scale. Larger scale reactions give more reproducible results with slightly higher catalyst levels of about 2 mole percent. Generally about 1-10 mole % of the cyanide source is preferred.

The process is conducted with a molar excess, with respect to the enol ester, of a moderate base. By the term "moderate base" is meant a substance which acts as a base yet whose strength or activity as a base lies between that of strong bases such as hydroxides (which could cause hydrolysis of the enol ester) and that of weak bases such as bicarbonates (which would not function effectively). Moderate bases suitable for use in this embodiment include both organic bases such as tertiary amines and inorganic bases such as alkali metal carbonates and phosphates. Suitable tertiary amines include trialkylamines such as triethylamine, trialkanolamines such as triethanolamine, and pyridine. Suitable inorganic bases include potassium carbonate and trisodium phosphate.

The base is used in an amount of from about 1 to about 4 moles per mole of enol ester, preferably about 2 moles per mole.

When the cyanide source is an alkali metal cyanide, particularly potassium cyanide, a phase transfer catalyst may be included in the reaction. Particularly suitable phase transfer catalysts are the Crown ethers.

A number of different solvents may be usable in this process, depending on the nature of the acid chloride or the acylated product. A preferred solvent for this reaction is 1,2-dichloroethane. Other solvents which may be employed, depending on the reactants or products include toluene, acetonitrile, methylene chloride, ethyl acetate, dimethylformamide, and methyl isobutyl ketone (MIBK).

In general, depending on the nature of the reactants and the cyanide source, the rearrangement may be conducted at temperatures up to about 50°C.

The above described substituted benzoyl chlorides can be prepared from the corresponding substituted benzoic acids according to the teaching of Reagents for Organic Synthesis, Vol. I, L.F. Fieser and M. Fieser, pp. 767-769 (1967). 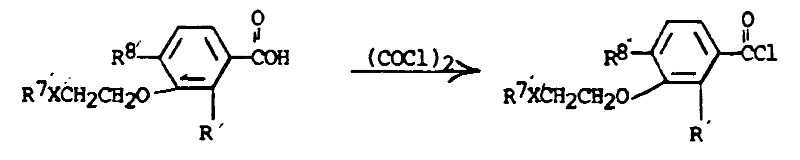
wherein X, R, R⁷ and R⁸ are as previously defined.

The above-described 5-hydroxy-4,4,6,6-tetra-substituted 1,3-cyclohexanediones can be prepared according to reaction (d). 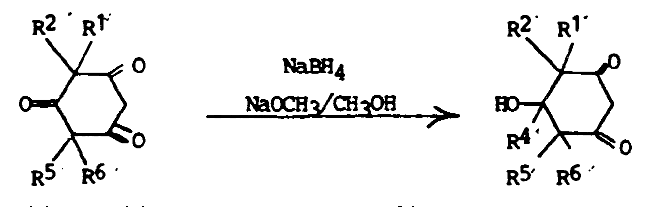
wherein R^{1'}, R^{1'}, R^{5'} and R^{6'} are as defined and R^{4'} is hydrogen.

In reaction (d) NaBH₄ is added to a basic methanolic solution of the syncarpic acid under controlled conditions and reacted at room temperature. The reaction solution is acidified and the product recovered by conventional techniques.

When R^{7'} is C₁-C₄ alkyl, C₂-C₅ alkenyl, C₂-C₅ alkynyl or cyano, the dione can be prepared by reacting a nucleophile such as methyl lithium with a 4,4,6,6-tetra-substituted 1,3,5-cyclohexanetrione as displayed in reaction (e). 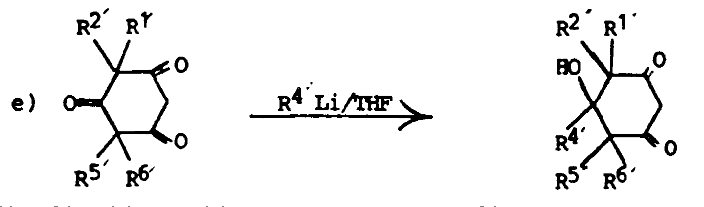
wherein R^{1'}, R^{2'}, R^{4'}, R^{5'} and R^{6'} are as defined and R^{4'} is C₁-C₄ alkyl, C₂-C₅ alkenyl, C₂-C₅ alkynyl or cyano.

In reaction (e) the lithium compound is added to a solution of the syncarpic acid reactant under controlled conditions and reacted at room temperature. The reaction solution is then acidified and the product recovered by conventional techniques.

The substituted 1,3-cyclohexanediones of the formula 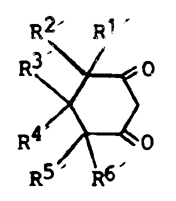
where R^{1'} through R^{5'} are as defined and R^{6'} is C₁-C₄ alkylthio or C₁-C₄ alkylsulfonyl can be prepared by a wide variety of general methods according to the teachings of Modern Synthetic Reactions, 2nd Edition, Chapter 9, H.O. House, W.A. Benjamin, Inc., Menlo Park, CA (1972).

The following is a table of certain selected compounds that are preparable according to the procedure described hereto. Compound numbers are assigned to each compound and are used throughout the remainder of the application.

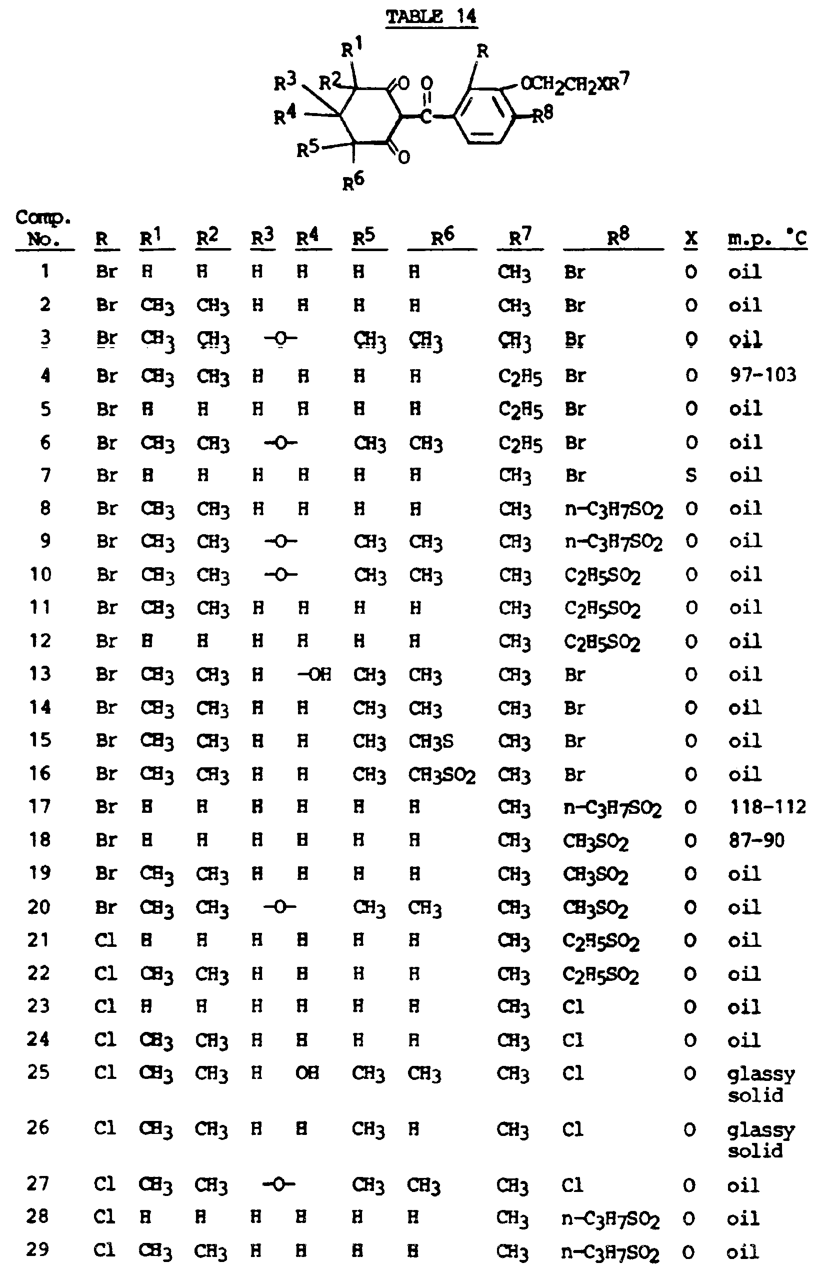

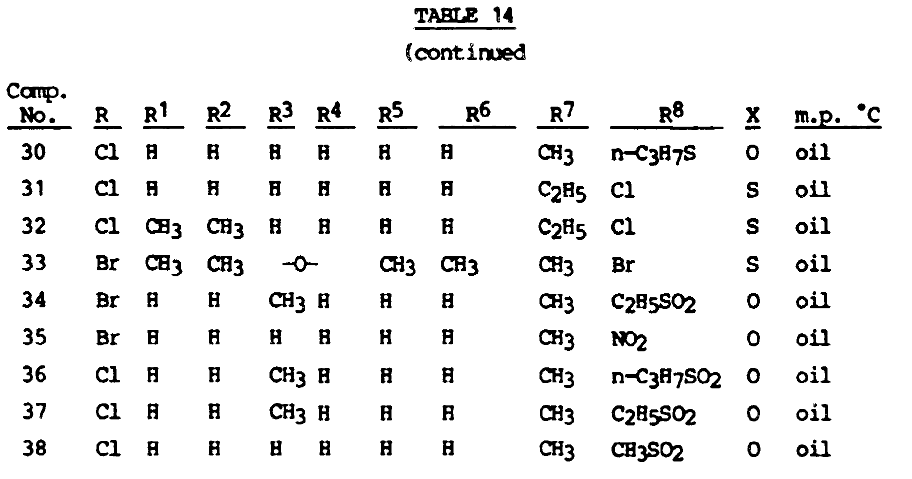

### Herbicidal Screening Tests

As previously mentioned, the herein described compounds produced in the above-described manner are phytotoxic compounds which are useful and valuable in controlling various plant species. Selected compounds of this invention were tested as herbicides in the following manner.

Pre-emergence multi-weed herbicide test. On the day preceding treatment, seeds of twelve different weed species are planted in loamy sand soil in individual rows using one species per row across the width of a flat. The weeds used are green foxtail (FT) (Setaria viridis), annual ryegrass (ARG) (Lolium multiflorum), watergrass (WG) (Echinochloa crusgalli), shattercane (SHC) (Sorghum bicolor), wild oat (WO) (Avena fatua), broadleaf signalgrass (BSG) (Brachiaria platyphylla), annual morningglory (AMG) (Ipomoea lacunosa), hemp sesbania (SESB) (Sesbania exaltata), velvetleaf (VL) (Abutilon theophrasti), sicklepod (SP) (Cassia obtusifolia, yellow nutsedge (YNG) (Cyperus esculentus) and cocklebur (CB) (Xanthium sp.). Ample seeds are planted to give about 20 to 40 seedlings per row, after emergence, depending upon the size of the plants.

Using an analytical balance, 37.5 milligrams (mg) of the compound to be tested are weighed out on a piece of glassine weighing paper. The paper and compound are placed in a 60 milliliter (ml) wide-mouth clear bottle and dissolved in 45 ml of acetone or substituted solvent. Eighteen ml of this solution are transferred to a 60 ml wide-mouth clear bottle and diluted with 22 ml of a water and acetone mature (19:1) containing enough polyoxyethylene sorbitan monolaurate emulsifier to give a final solution of 0.5% (v/v). The solution is then sprayed on a seeded flat on a linear spray table calibrated to deliver 40 gallons per acre (748 L/ha). The application rate is/14 lb/acre (0.28 Kg/ha).

After treatment, the flats are placed in the greenhouse at a temperature of 70 to 80°F and watered by sprinkling. Two weeks after treatment, the degree of injury or control is determined by comparison with untreated check plants of the same age. The injury rating from 0 to 100% is recorded for each species as percent control with 0% representing no injury and 100% representing complete control.

The results of the test are shown in the following Table 15.

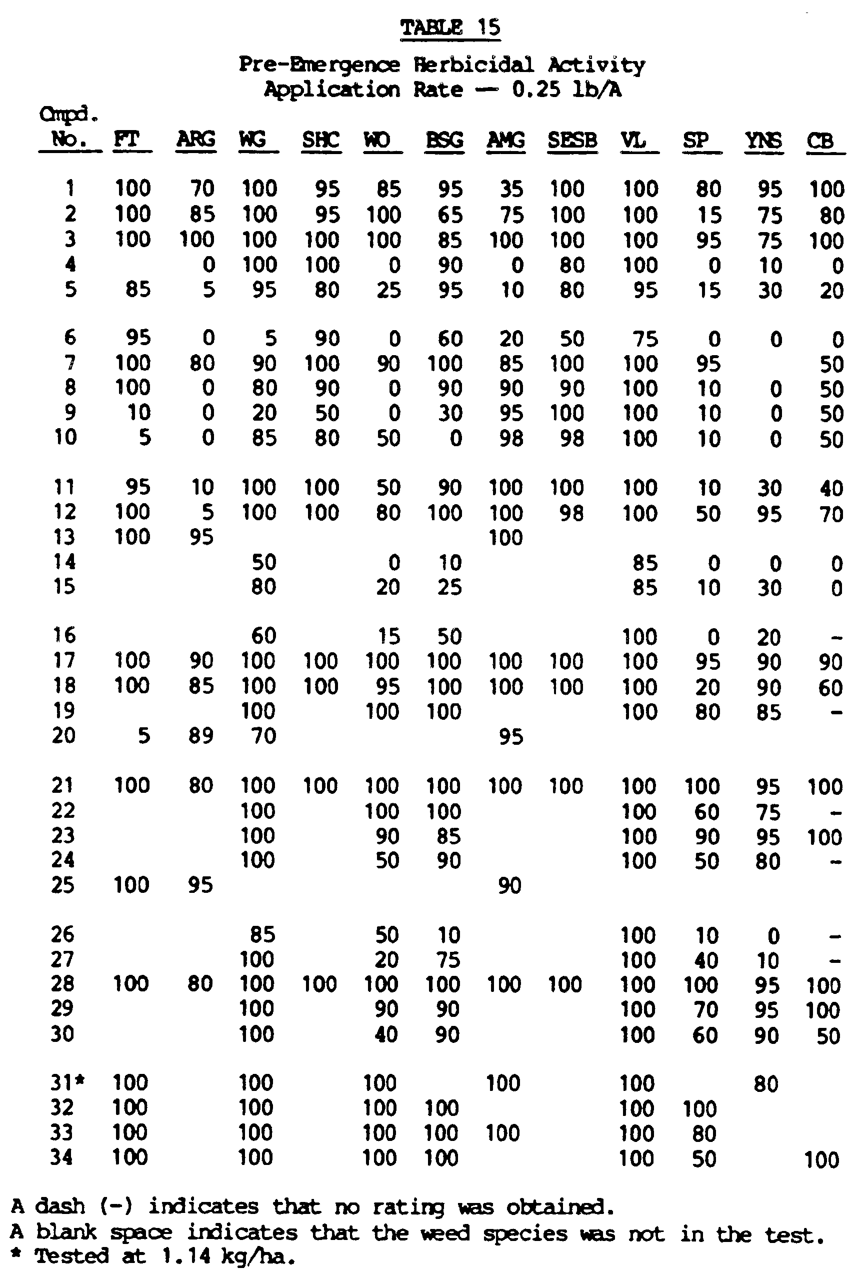

Post-Emergence Multi-Weed Herbicide Test: This test is conducted in an identical manner to the testing procedure for the pre-emergence multi-weed herbicide test, except the seeds of the twelve different weed species are planted 10-12 days before treatment. Also, watering of the treated flats is confined to the soil surface and not to the foliage of the sprouted plants.

Post-Emergence Multi-Weed Herbicide Test: This test is conducted in an identical manner to the testing procedure for the post-emergence herbicide test, except the seeds of the twelve weed species used in the pro-emergence multi-weed herbicide test were used and the seeds were planted 10-12 days before treatment. Also, watering of the treated flats is confined to the soil surface and not to the foliage of the sprouted plants.

The results of the post-emergence multi-weed herbicide test are reported in Table 16.

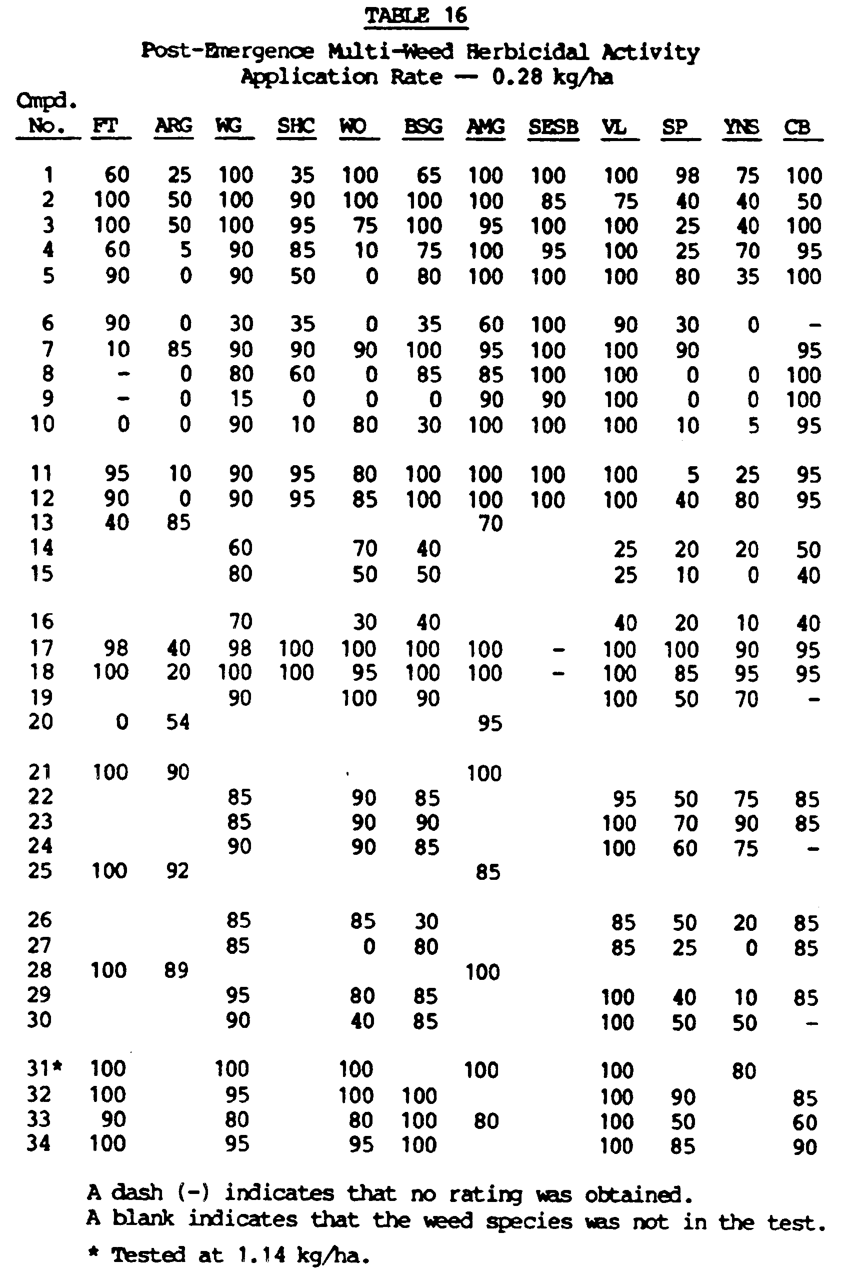

## Claims (Claims for the following Contracting State(s): ES)

1. A process for preparing a compound of formula (d) 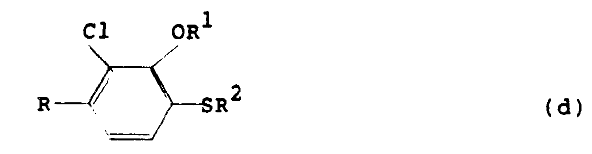 where R is cyano, carboxy or CO₂R^{a} where R^{a} is C₁₋₄ alkyl, R¹ is C₁₋₄ alkyl, C₁₋₄ haloalkyl, - CH₂CH₂OCH₃, - CH₂CH₂OC₂H₅, -CH₂CH₂SCH₃ or CH₂CH₂SC₂H₅, and R² is C₁₋₄ alkyl;
which process comprises reacting a compound of formula (f) 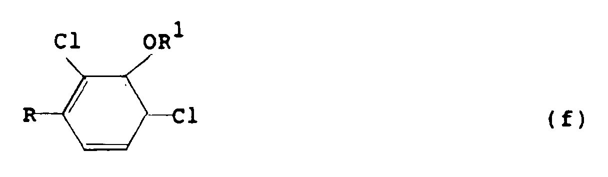 with a mercaptan of formula R²SH in the presence of base.

2. A process according to claim 1 wherein the base is a molar excess of potassim carbonate.

3. A process according to claim 1 or claim 2 wherein the reaction is effected in dimethylformanide as solvent.

4. A process according to any one of claims 1 to 3 wherein the reaction is a temperature of from 50°C to 100°C.

5. A process according to anyone of claim 1 to 4 wherein the compound of formula (d) is subsequently either;
(a) oxidized to form a compound of formula 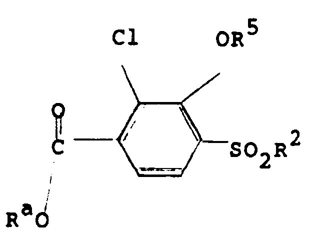 where R^{a} and R² are as defined in claim 1 and R⁵ is C₁₋₄ alkyl, C₁₋₄ haloalkyl, CH₂CH₂OCH₃, or CH₂CH₂OC₂H₅; and thereafter if desired hydrolysed to a compound of formula 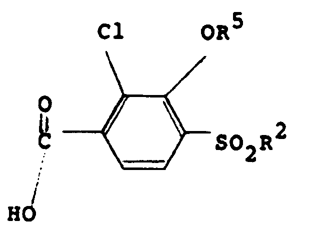 or
(b) hydrolysed to a compound of formula.

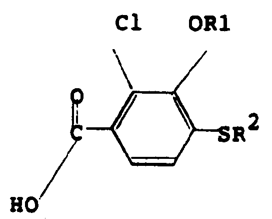

6. A process according to any one of claims 1 to 5 wherein the compound of formula (f) is prepared by reacting a compound of formula 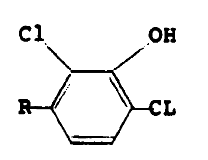 wherein R is as defined in claim 1 with an alkylating agent in the presence of a base.

## Claims (Claims for the following Contracting State(s): GR)

1. A compound of structural formula (a), (b), (c), (d) or (e) 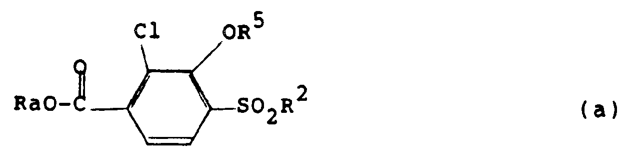 where Rₐ is C₁₋₄alkyl; R⁵ is C₁₋₄alkyl, C₁₋₄haloalkyl, CH₂CH₂OCH₃ or CH₂CH₂OC₂H₅; and R² is C₁₋₄alkyl; 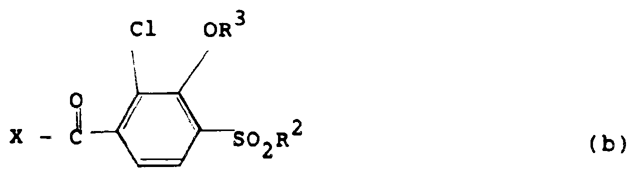 where R² is as defined above; R³ is -CH₂CH₂OCH₃ or -CH₂CH²OC₂H₅; and X is hydroxy or chlorine, provided that when X is hydroxy, R² is not methyl; 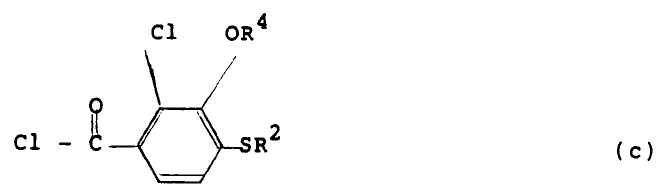 where R² is as defined above and R⁴ is -CH₂CH₂OCH₃, -CH₂CH₂SCH₃ or - CH₂CH₂SC₂H₅; 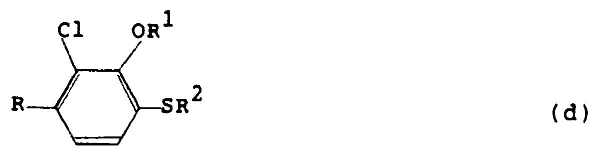 where R is cyano, carboxy or CO₂R^{a} where Rₐ is as defined above, R¹ is C₁₋₄ alkyl, C₁₋₄haloalkyl, - CH₂ CH₂OCH₃, -CH₂CH₂OC₂H₅, -CH₂CH₂SCH₃ or CH₂CH₂SC₂H₅, and R² is as defined above; with the proviso that when R is carboxyl, then R¹ is -CH₂CH₂OCH₃, -CH₂CH₂OC₂H₅, -CH₂CH₂SCH₃ or - CH₂CH₂SC₂H₅; and 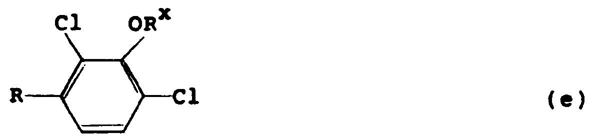 where R is as defined above and Rₓ is hydrogen or R¹ where R¹ is as defined above, with the proviso that when R is carboxy and R^{x} is a group R¹, the group R¹ is -CH₂CH₂OCH₃, - CH₂CH₂OC₂H₅, -CH₂ CH₂SCH₃ or -CH₂CH₂SC₂H₅.

2. A compound according to claim 1 of formula (a) where R^{a} is ethyl; R⁵ is CH₂CH₂OCH₃ or - CH₂CH₂OC₂H₅ and R² is C₁₋₃ alkyl.

3. A compound according to claim 1 of formula (b) where X is hydroxy or chlorine; R³ is - CH₂CH₂OCH₃ and R² is C₁₋₄ alkyl.

4. A compound acording to claim 1 or claim 3 of formula (b) where X is chlorine.

5. A compound according to claim 1 or claim 3 of formula (b) where X is hydroxy.

6. A compound according to claim 1 of formula (c) where R⁴ is -CH₂CH₂OCH₅ and R² is C₁₋₄alkyl.

7. A compound according to claim 1 of formula (d) wherein R is carboethoxy, R' is C₁₋₃ alkyl, - CH₂CH₂OCH₃, -CH₂CH₂OC₂H₅ or -CH₂CH₂SCH₃.

8. A compound according to claim 1 of formula (e) wherein R carboethoxy or carboxy and R^{x} is R¹ wherein R¹ is -CH₂CH₂OCH₃, -CH₂CH₂OC₂H₅ or -CH₂CH₂SCH₃.

9. A compound according to claim 1 of formula (e) wherein R is carboethoxy or carboxy and R¹ is C₁₋₄alkoxy, -CH₂CH₂OCH₃, -CH₂CH₂OC₂H₅ or -CH₂CH₂SCH₃.

10. A compound according to claim 1 of formula (e) where R is carbomethoxy or carboethoxy and R^{x} is R¹ which is CH₂CH₂OCH₃.

11. A compound according to claim 1 of formula (e) wherein R^{x} is hydrogen and R is carbomethoxy, carboethoxy, cyano or carboxy.

12. A compound according to claim 1 of formula (e) where R^{x} is hydrogen and R is carboethoxy.

13. A process for preparing a compound of formula (d) 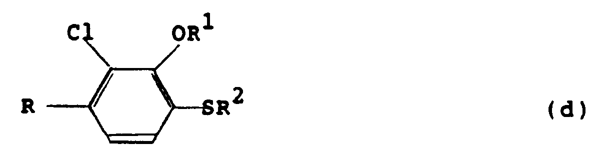 where R is cyano, carboxy or CO₂R^{a} where Ra is C₁₋₄ alkyl, R¹ is C₁₋₄ alkyl, C₁₋₄ haloalkyl, - CH₂CH₂OCH₃, -CH₂CH₂OC₂H₅, -CH₂CH₂SCH₃ or CH₂CH₂SC₂H₅, and R² is C₁₋₄ alkyl; which process comprises reacting a compound of formula (f) 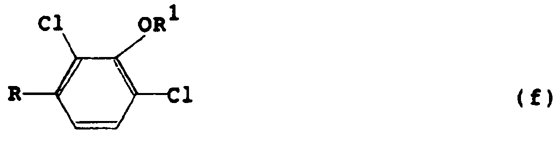 with a mercaptan of formula R²SH in the presence of a base.

## Patentansprüche (Patentansprüche für folgende(n) Vertragsstaat(en): ES)

1. Verfahren zur Herstellung einer Verbindung der Formel (d) 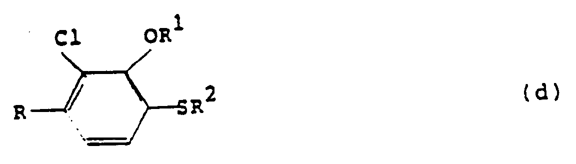 Worin R für Cyano, Carbocy oder CO₂R^{a} steht, wobei R^{a} C₁₋₄-Alkyl bedeutet; R¹ für C₁₋₄-Alkyl, C₁₋₄-Halogennoalkyl, -CH₂CH₂OCH₃, -CH₂CH₂OC₂H₅, -CH₂CH₂SCH₃ oder -CH₂CH₂SC₂H₅ steht; und R² für C₁₋₄-Alkyl steht; bei welchem Verfahren eine Verbindung der Formel (f) 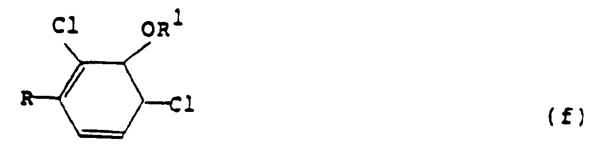 mit einem Mercaptan der Forem R²SH in Gegenwart einer Base umgesetzt wird.

2. Verfahren nach Anspruch 1, bei welchem als Base Kaliumcarbonat in einem molaren Überschuß verwendet wird.

3. Verfahren nach Anspruch 1 oder 2, bei welchem die Reaktion in Dimethylformamid als Lösungsmittel ausgeführt wird.

4. Verfahren nach einem der Anspürche 1 bis 3, bei welchem die Reaktion bei einer Temperatur von 50 bis 100°C ausgeführt wird.

5. Verfahren nach einem der Anspürche 1 bis 4, bei welchem die Verbindung der Formel (d) anschließend entweder
(a) oxidiert wird, um eine Verbindung der Formel 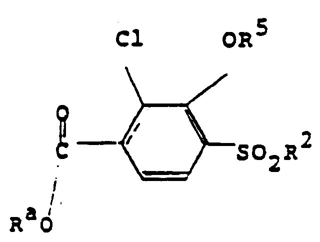 herzustellen, worin R^{a} und R² die in Anspruch 1 angegebenen Bedeutungen besitzen und R⁵ für C₁₋₄-Alkyl, C₁₋₄-Halogenoalkyl, -CH₂CH₂OCH₃ oder -CH₂CH₂OC₂H₅ steht, worauf sie gegebenenfalls zu einer Verbindung der Formel 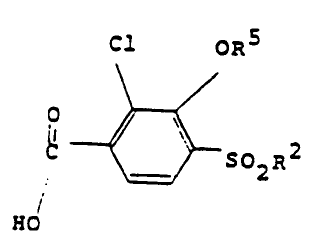 hydrolysiert wird; oder
(b) hydrolysiert wird, um eine Verbindung der Formel 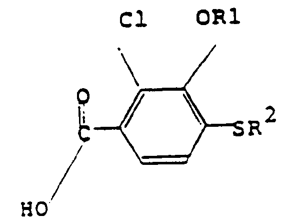 herzustellten.

6. Verfahren nach einem der Ansprüche 1 bis 5, bei welchem die Verbindung der Formel (f) dadurch hergestellt wird, daß eine Verbindung der Formel 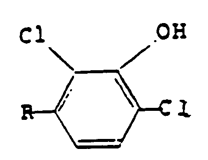 worin R die in Anspruch 1 angegebene Bedeutung besitzt,mit einem Alkylierungsmittel in Gegenwart einer Base umgesetzt wird.

## Patentansprüche (Patentansprüche für folgende(n) Vertragsstaat(en): GR)

1. Verbindung mit der Strukturformel (a), (b), (c), (d) oder (e) 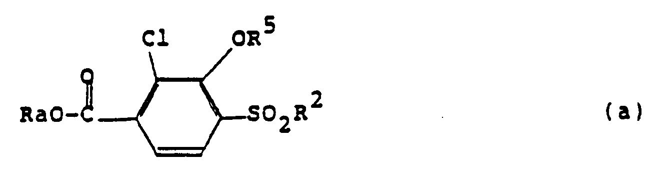 in der Rₐ für C₁₋₄-Alkyl, R⁵ für C₁₋₄-Alkyl, C₁₋₄-Haloalkyl, CH₂CH₂OCH₃ oder CH₂CH₂OC₂H₅ und R² für C₁₋₄-Alkyl steht, 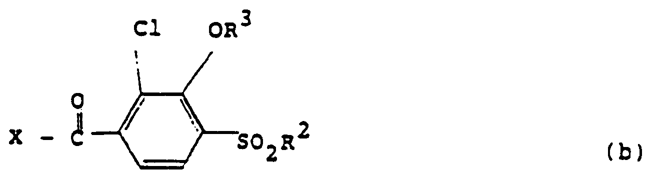 in der R² wie oben definiert ist, R³ für -CH₂CH₂OCH₃ oder -CH₂CH₂OC₂H₅ und X für Hydroxy oder Chlor steht, mit der Maßgabe, daß R² nicht für Methyl steht, wenn X für Hydroxy steht, 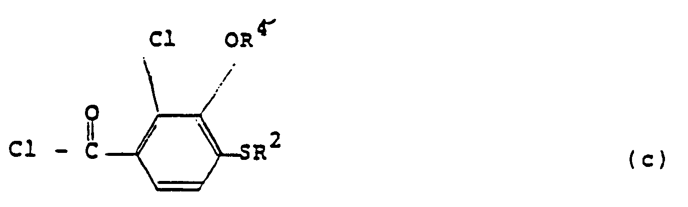 in der R² wie oben definiert ist und R⁴ für -CH₂CH₂OCH₃, -CH₂CH₂SCH₃ oder -CH₂CH₂SC₂H₅ steht 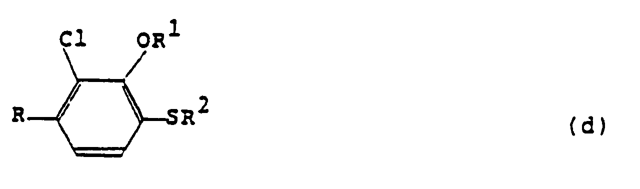 in der R für Cyano, Carboxy oder CO₂R^{a} steht, wobei R^{a} wie oben definiert ist, R¹ für C₁₋₄-Alkyl, C₁₋₄-Haloalkyl, -CH₂CH₂OCH₃, -CH₂CH₂OC₂H₅, -CH₂CH₂SCH₃ oder CH₂CH₂SC₂H₅ steht und R² wie oben definiert ist, mit der Maßgabe, daß R¹ für -CH₂CH₂OCH₃, -CH₂CH₂OC₂H₅, -CH₂CH₂SCH₃ oder -CH₂CH₂SC₂H₅ steht, wenn R für Carboxy steht, und 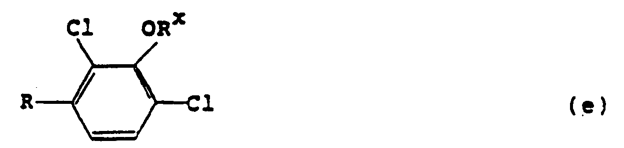 in der R wie oben definiert ist und Rₓ für Wasserstoff oder R¹ steht, wobei R¹ wie oben definiert ist, mit der Maßgabe, daß die Gruppe R¹ für -CH₂CH₂OCH₃, -CH₂CH₂OC₂H₅, -CH₂CH₂SCH₃ oder -CH₂CH₂SC₂H₅ steht, wenn R für Carboxy und R^{x} für eine Gruppe R¹ steht.

2. Verbindung nach Anspruch 1 der Formel (a), worin R^{a} für Ethyl steht; R⁵ für - CH₂CH₂OCH₃ oder -CH₂ CH₂OC₂H₅ steht; und R² für C₁₋₃-Alkyl steht.

3. Verbindung nach Anspruch 1 der Formel (b), worin X für Hydroxy oder Chlor steht; R³ für - CH₂CH₂OCH₃ steht; und R² für C₁₋₄-Alkyl steht.

4. Verbindung nach Anspruch 1 oder 3 der Formel (b), worin X für Chlor steht.

5. Verbindung nach Anspruch 1 oder 3 der Formel (b), worin X für Hydroxy steht.

6. Verbindung nach Anspruch 1 der Formel (c), worin R⁴ für -CH₂CH₂OCH₃ und R² für C₁₋₄-Alkyl steht.

7. Verbindung nach Anspruch 1 der Formel (d), worin R für Carboethoxy und R¹ für C₁₋₃-Alkyl,-CH₂ CH₂OCH₃, -CH₂CH₂OC₂H₅ oder -CH₂CH₂SCH₃ steht.

8. Verbindung nach Anspruch 1 der Formel (e), worin R für Carboethoxy oder Carboxy steht; und R^{x} für R¹ steht, wobei R¹ -CH₂CH₂OCH₃, -CH₂CH₂OC₂H₅ oder -CH₂CH₂SCH₃ bedeutet.

9. Verbindung nach Anspruch 1 der Formel (e), worin R für Carboethoxy oder Carboxy und R¹ für C₁₋₄-Alkyl, -CH₂CH₂OCH₃, -CH₂CH₂OC₂H₅ oder -CH₂CH₂SCH₃ steht.

10. Verbindung nach Anspruch 1 der Formel (e), worin R für Carbomethoxy oder Carboethoxy steht; und R^{x} für R¹ steht, wobei R¹ -CH₂CH₂OCH₃ bedeutet.

11. Verbindung nach Anspruch 1 der Formel (e), worin R^{x} für Wasserstoff und R für Carbomethoxy, Carboethoxy, Cyano oder Carboxy steht.

12. Verbindung nach Anspruch 1 der Formel (e), worin R^{x} für Wasserstoff und R für Carboethoxy steht.

13. Verfahren zur Herstellung einer Verbindung der Formel (d) 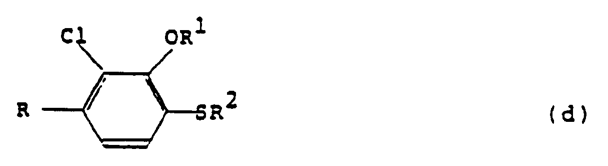 worin R für Cyano, Carboxy oder CO₂R^{a} steht, wobei R^{a} C₁₋₄-Alkyl bedeutet; R¹ für C₁₋₄-Alkyl, C₁₋₄-Halogenoalkyl, -CH₂CH₂OCH₃, -CH₂CH₂OC₂H₅, -CH₂CH₂SCH₃ oder -CH₂CH₂SC₂H₅ steht; und R² für C₁₋₄-Alkyl steht; bei welchem eine Verbindung der Formel (f) 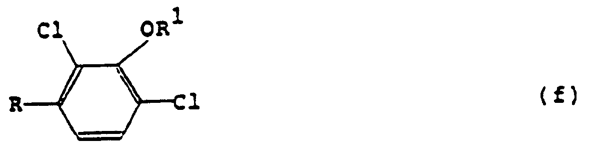 mit einem Mercaptan der Formel R²SH in Gegenwart einer base umgesetzt wird.

## Revendications (Revendications pour l'(les) Etat(s) contractant(s) suivant(s): ES)

1. Procédé de préparation d'un composé de formule (d) 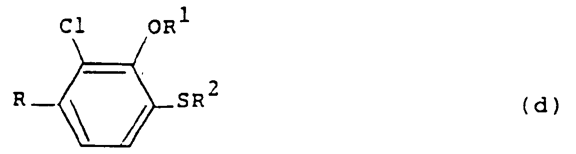 dans laquelle R est un groupe cyano, carboxy ou CO₂R^{a}, où R^{a} est un groupe alkyle en C₁ à C₄, R¹ est un groupe alkyle en C₁ à C₄, halogénalkyle en C₁ à C₄, -CH₂CH₂OCH₃, - CH₂CH₂OC₂H₅, -CH₂CH₂SCH₃ ou -CH₂CH₂SC₂H₅ et R² est un groupe alkyle en C₁ à C₄;
procédé qui consiste à faire réagir un composé de formule (f) 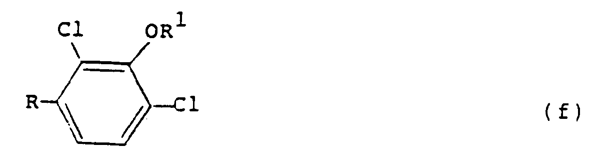 avec un mercaptan de formule R²SH en présence d'une base.

2. Procédé suivant la revendication 1, dans lequel la base est un excès molaire de carbonate de potassium.

3. Procédé suivant la revendication 1 ou la revendication 2, dans lequel la réaction est conduite dans du diméthylformamide comme solvant.

4. Procédé suivant l'une quelconque des revendications 1 à 3, dans lequel la réaction est conduite à une température de 50°C à 100°C.

5. Procédé suivant l'une quelconque des revendications 1 à 4, dans lequel le composé de formule (d) est ensuite:
(a) oxydé pour former un composé de formule 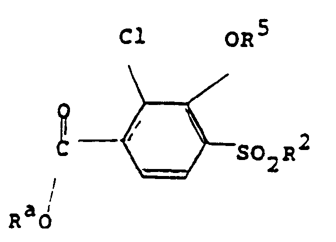 dans laquelle R^{a} et R² sont tels que définis dans la revendication 1 et R⁵ est un groupe alkyle en C₁ à C₄, halogénalkyle en C₁ à C₄, -CH₂CH₂OCH₃ ou -CH₂CH₂OC₂H₅ ; puis, le cas échéant, hydrolysé en un composé de formule 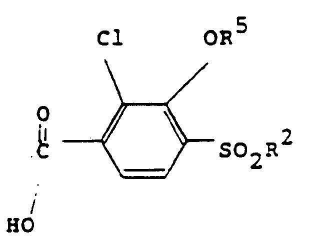 ou bien
(b) hydrolysé en un composé de formule

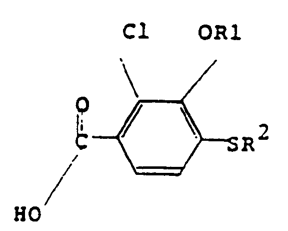

6. Procédé suivant l'une quelconque des revendications 1 à 5, dans lequel le composé de formule (f) est préparé par réaction d'un composé de formule 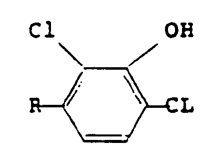 dans laquelle R est tel que défini dans la revendication 1, avec un agent alkylant en présence d'une base.

## Revendications (Revendications pour l'(les) Etat(s) contractant(s) suivant(s): GR)

1. Composé de formule structurale (a), (b), (c), (d) ou (e) 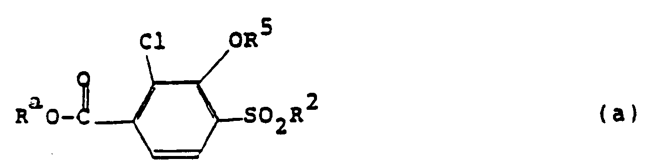 dans laquelle R^{a} représente un groupe alkyle en C₁ à C₄ ; R⁵ représente un groupe alkyle en C₁ à C₄, halogénalkyle en C₁ à C₄, CH₂CH₂OCH₃ ou CH₂CH₂OC₂H₅ ; et R² représente un groupe alkyle en C₁ à C₄ ; 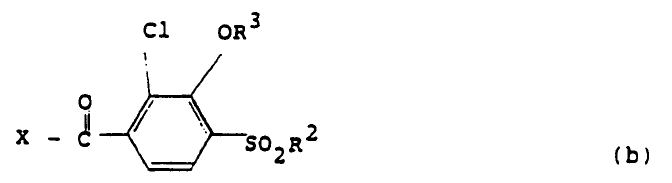 dans laquelle R² répond à la définition précitée ; R³ représente un groupe CH₂CH₂OCH₃ ou CH₂CH₂OC₂H₅ ; et X représente un groupe hydroxy ou chloro ; sous réserve que, lorsque X représente un groupe hydroxy, R² ne représente pas un groupe méthyle ; 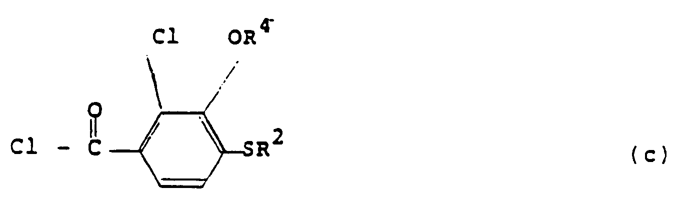 dans laquelle R² répond à la définition précitée et R⁴ représente un groupe -CH₂CH₂OCH₃, -CH₂CH₂SCH₃ ou -CH₂CH₂SC₂H₅ ; 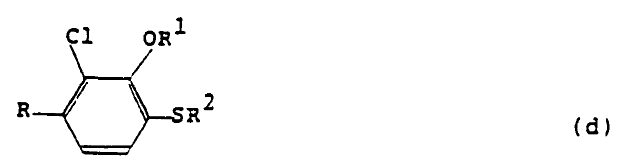 dans laquelle R représente un groupe cyano, carboxy ou CO₂R^{a} dans lequel R^{a} répond à la définition précitée, R¹ représente un groupe alkyle en C₁ à C₄, halogénalkyle en C₁ à C₄, -CH₂CH₂OCH₃, -CH₂CH₂OC₂H₅, -CH₂CH₂SCH₃ ou -CH₂CH₂SC₂H₅, et R² répond à la définition précitée ; sous réserve que, lorsque R représente un groupe carboxyle, R¹ représente alors un groupe -CH₂CH₂OCH₃, -CH₂CH₂OC₂H₅, -CH₂CH₂SCH₃ ou -CH₂CH₂SC₂H₅ ; et 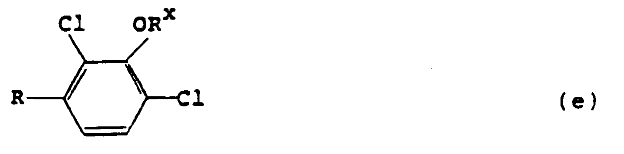 dans laquelle R répond à la définition précitée et R^{x} représente l'hydrogène ou un groupe R¹, le groupe R¹ répondant à la définition précitée, sous réserve que, lorsque R représente un groupe carboxy et R^{x} représente un groupe R¹, le groupe R¹ consiste en un groupe -CH₂CH₂OCH₃, -CH₂CH₂OC₂H₅, -CH₂CH₂SCH₃ ou -CH₂CH₂SC₂H₅.

2. Composé suivant la revendication 1, de formule (a) dans laquelle R^{a} est un groupe éthyle; R⁵ est un groupe -CH₂CH₂OCH₃ ou -CH₂CH₂OC₂H₅ et R² est un groupe alkyle en C₁ à C₃.

3. Composé suivant la revendication 1 de formule (b) dans laquelle X est un groupe hydroxy ou du chlore : R³ est un groupe -CH₂CH₂OCH₃ et R² est un groupe alkyle en C₁ à C₄.

4. Composé suivant la revendication 1 ou la revendication 3 de formule (b) dans laquelle X est le chlore.

5. Composé suivant la revendication 1 ou la revendication 3, de formule (b) dans laquelle X est un groupe hydroxy.

6. Composé suivant la revendication 1, de formule (c) dans laquelle R⁴ est un groupe - CH₂CH₂OCH₅ et R² est un groupe alkyle en C₁ à C₄.

7. Composé suivant la revendication 1, de formule (d) dans laquelle R est un groupe carbéthoxy, R¹ est un groupe alkyle en C₁ à C₃, -CH₂CH₂OCH₃, -CH₂CH₂OC₂H₅ ou -CH₂CH₂SCH₃.

8. Composé suivant la revendication 1, de formule (e) dans laquelle R est un groupe carbéthoxy ou carboxy et R^{x} repréente R¹, R¹ étant un groupe -CH₂CH₂OCH₃, -CH₂CH₂OC₂H₅ ou - CH₂CH₂SCH₃.

9. Composé suivant la revendication 1, de formule (e) dans laquelle R est un groupe carbéthoxy ou carboxy et R¹ est un groupe alkoxy en C₁ à C₄, -CH₂CH₂OCH₃, -CH₂CH₂OC₂H₅ ou - CH₂CH₂SCH₃.

10. Composé suivant la revendication 1, de formule (e) dans laquelle R est un groupe carbométhoxy ou carbéthoxy et R^{x} représente R¹, qui est un groupe -CH₂CH₂OCH₃.

11. Composé suivant la revendication 1, de formule (e) dans laquelle R^{x} est l'hydrogène et R est un carbométhoxy, carbéthoxy, cyano ou carboxy.

12. Composé suivant la revendication 1, de formule (e) dans laquelle R^{x} est l'hydrogène et R est un groupe carbéthoxy.

13. Procédé de préparation d'un composé de formule (d) 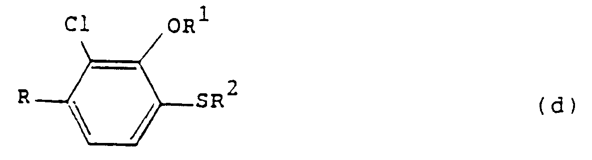 dans laquelle R est un groupe cyano, carboxy ou CO₂R^{a}, où R^{a} est un groupe alkyle en C₁ à C₄, R¹ est un groupe alkyle en C₁ à C₄, halogénalkyle en C₁ à C₄, -CH₂CH₂OCH₃, - CH₂CH₂OC₂H₅, -CH₂CH₂SCH₃ ou -CH₂CH₂SC₂H₅ et R² est un groupe alkyle en C₁ à C₄ ;
procédé qui consiste à faire réagir un composé de formule (f) 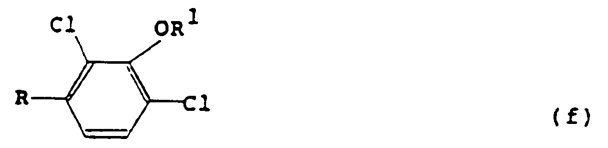 avec un mercaptan de formule R²SH en présence d'une base.
